# EUROPEAN PATENT APPLICATION

(11) **EP 4 159 233 A1**
(43) Date of publication of application: **05.04.2023**
(21) Application number: 21812811.4
(22) Date of filing: 26.05.2021
(51) Int. Cl.: A61K 39/215, A61K 39/39, A61P 31/14

(54) **PHARMACEUTICAL COMPOSITION COMPRISING POLYNUCLEOTIDES AND USE THEREOF FOR PREVENTION OR TREATMENT OF COVID-19**

(30) Priority: 29.05.2020 CN 202010475388
(71) Applicant: Beijing Yisheng Biotechnology Co., Ltd., Beijing, Daxing District 102600 (CN); Liaoning Yisheng Biopharma Co., Ltd., Liaoning 110131 (CN)
(72) Inventor: ZHANG, Yi, Beijing 102600 (CN); LIU, Yuan, Beijing 102600 (CN); ZHANG, Nan, Beijing 102600 (CN)
(74) Representative: Hirsch & Associés
(86) International application number: PCT/CN2021/096048
(87) International publication number: WO 2021/238982

(57) **Abstract**

The present application relates to a pharmaceutical composition comprising polynucleotides and use thereof for prevention or treatment of COVID-19. More specifically, disclosed in the present application is a composition used for prevention or treatment of COVID-19, comprising a polyriboinosinic-polyribocytidylic acid, an antibiotic or polyamino compound, a positive ion, and an optional antigen derived from novel coronavirus SARS-CoV-2. Also provided is use of the composition in preparation of a drug or vaccine for prevention or treatment of novel coronavirus SARS-CoV-2.

## Description

The present disclosure claims the priority to the Chinese patent application "Pharmaceutical Composition Comprising Polynucleotides and Use thereof for Prevention or Treatment of COVID-19" (filing No. 202010475388.6) filed on May 29, 2020.

### TECHNICAL FIELD

The present disclosure relates to the field of medical science, and particularly to prevention or treatment of coronavirus infection. More specifically, the present disclosure relates to a pharmaceutical composition for prevention or treatment of coronavirus infection, which contains a polyriboinosinic-polyribocytidylic acid (PIC), an antibiotic (or polyamino compound), a positive ion, and an optional antigen derived from virus (for example, but not limited to: virus particles, proteins, nucleic acids, peptides), and use of the composition for prevention or treatment of coronavirus infection.

### BACKGROUND ART

The novel coronavirus infection is mainly caused by novel coronavirus (SARS-CoV-2, also referred to as 2019-nCOV). The route of infection is mainly respiratory droplet transmission, and also may be contact transmission. Humans are generally susceptible. The aged and those with underlying diseases are more seriously ill after being infected, and children and infants are also affected.

Based on current epidemiological survey, COVID-19 has latency of 1-14 days, mostly 3-7 days, and is mainly manifested by fever, dry cough, and hypodynamia. A minority of patients are accompanied by symptoms such as nasal obstruction, runny nose, sore throat, myalgia, and diarrhea. Severe patients often have symptoms of dyspnea and/or hypoxemia one week after onset, and in serious cases, the patients may rapidly develop acute respiratory distress syndrome, septic shock, uncorrectable metabolic acidosis, bleeding and coagulation dysfunctions and multiple organ failure, etc. It is noteworthy that the severe and critically ill patients may have moderate to low fever or even no obvious fever during the course of disease. Mild patients manifest only low fever, slight hypodynamia and the like, without manifestation of pneumonia. Judging from the situation of current cases, most patients have good prognosis, and a few patients are in critical condition. The aged and those with chronic underlying diseases have poor prognosis. Child cases have relatively mild symptoms. The source of infection seen so far is mainly patients infected with the novel coronavirus. Asymptomatic carriers may also become the infection source. The virus has a strong transmission ability, and there is no effective medicine, thus it is extremely harmful.

For the treatment of COVID-19, no effective anti-viral treatment method is currently identified. Most candidate drugs are only limited to *in vitro* experiments, and the therapeutic effects thereof are not verified in animals or human bodies. Accordingly, there is still a need in the art to provide an effective composition for anti-viral treatment.

### SUMMARY

### Immune composition

In view of this, the present disclosure provides an immune composition, which contains polyriboinosinic-polyribocytidylic acid (polyLC, PIC), an antibiotic (or polyamino compound), a positive ion, and an optional antigen derived from a virus; or, consists of PIC, an antibiotic (or polyamino compound), a positive ion, and an optional viral antigen.

In some embodiments, the virus is a coronavirus, such as SARS-CoV-2 (covering variants or subspecies thereof). In some embodiments, the virus is inactivated and crude or inactivated and purified.

In some embodiments, vaccines or immune compositions suitable for the present disclosure include, but are not limited to, inactivated type, subunit type, genetic recombination type, polypeptide type vaccines, and nucleic acid type.

In some embodiments, the PIC suitable for the present disclosure may be a commercially available PIC, or a polyinosinic acid-polycytidylic acid-based adjuvant disclosed in the art (for example, 2006800007767 and 2006800010596), which are incorporated herein by reference in entirety.

In some embodiments, the PIC in the immune composition is heterogeneous in molecular weight, and has a molecular weight at least 50,000 daltons. For example, the numerical value of 66,000 daltons is equivalent to the molecular size of 6.4S sedimentation coefficient units (Svedbergs). In some embodiments, the PIC is 66,000 daltons to 1,200,000 daltons (equivalent to 6.4 to 24.0 sedimentation coefficient units). In some other embodiments, the molecular weight of the PIC is at least 150,000 daltons. In some other embodiments, the molecular weight of the PIC is 100,000 to 200,000 daltons, or 300,000 to 4,000,000 daltons, or 500,000 to 1,000,000 daltons, or 1,000,000 to 1,500,000 daltons, or 1,500,000 to 2,000,000 daltons, or 2,000,000 to 2,500,000 daltons, or 2,500,000 to 3,000,000 daltons, or 3,000,000 to 3,500,000 daltons, or 3,500,000 to 4,000,000 daltons, or 4,000,000 to 4,500,000 daltons, or 4,500,000 to 5,000,000 daltons.

The term "heterogeneous" used in the present disclosure means that the PIC molecules contained in the immune composition or vaccine are not homogeneous in the physical properties of molecular weight, size, or both.

In some embodiments, the concentration of the PIC in the immune composition/vaccine is 250 µg/unit dose to 5000 µg/unit dose; for example, the concentration of the PIC in the composition is selected from the group consisting of 250 µg/unit dose, 500 µg/unit dose, 1000 µg/unit dose, 1500 µg/unit dose, 2000 µg/unit dose, 3000 µg/unit dose, 4000 µg/unit dose, 5000 µg/unit dose, and a range between any two values of the foregoing.

In a specific embodiment, the concentration of the PIC in the immune composition/vaccine is 500 µg/unit dose to 4000 µg/unit dose, 1000 µg/unit dose to 3000 µg/unit dose, or 1000 µg/unit dose to 2500 µg/unit dose.

When the immune composition/vaccine of the present disclosure is applied to humans, the concentration of the PIC in the immune composition/vaccine is selected from the group consisting of 500 µg/unit dose, 1000 µg/unit dose, 1500 µg/unit dose, 2000 µg/unit dose, and a range between any two values of the foregoing. When the immune composition/vaccine of the present disclosure is applied to juveniles (e.g. children), the concentration of the PIC in the immune composition/vaccine is selected from the group consisting of 250 µg/unit dose, 500 µg/unit dose, 1000 µg/unit dose, 1250 µg/unit dose, and a range between any two values of the foregoing.

In a specific embodiment, the antibiotic contained is selected from the group consisting of tobramycin, anthracycline, butyrosin sulfate, gentamicin, hygromycin, amikacin, kanamycin (or dideoxykanamycin), nebramycin, metrzamide, neomycin, puromycin, streptomycin, streptozotocin, and a combination thereof. The polyamino compound is selected from the group consisting of spermidine acid salt, spermidine, N-(3-aminopropyl), N-(3-aminopropyl)-1,4-butanediamine, spermine, spermine, OS-dimethylamidothiophosphate, polylysine, aminoglycoside, and a combination thereof. In some embodiments, the concentration of the antibiotic in the composition is 10 units/ml to 100,000 units/ml, preferably 100 units/ml to 10,000 units/ml, more preferably 500 units/ml to 5,000 units/ml.

In some embodiments, the concentration of the antibiotic or polyamino compound in the immune composition may be mentioned as 400 U/unit dose to 1200 U/unit dose, for example, but not limited to 400, 500, 600, 700, 800, 900, 1000, 1100, 1200 U/unit dose.

In some other embodiments, the concentration of the antibiotic or polyamino compound in the immune composition may be mentioned as 400 U/ml to 1200 U/ml, for example, but not limited to 400, 500, 600, 700, 800, 900, 1000, 1100, 1200 U/ml.

In some embodiments, the positive ion contained is a divalent positive ion, and is selected from the group consisting of calcium, cadmium, lithium, magnesium, cerium, cesium, chromium, cobalt, deuterium, gallium, iodine, iron, zinc, and a combination thereof. In a specific embodiment, the positive ion is a calcium ion. The positive ion may be in a form of any suitable salt or organic complex, including, but not limited to, chloride, fluoride, hydroxide, phosphate or sulfate. For example, when the positive ion is calcium, the calcium ion may be in a form of calcium carbonate, calcium chloride, calcium fluoride, calcium hydroxide, calcium phosphate or calcium sulfate. In some embodiments, the concentration of the positive ion in the composition is 10 µmol to 10 mmol/ml, for example, 50 µmol to 5 mmol/ml; or 100 µmol to 1 mmol/ml.

In some embodiments, the concentration of the positive ion in the immune composition is 0.01 mg/unit dose to 0.1 mg/unit dose, for example, but not limited to, 0.01, 0.02, 0.03, 0.04, 0.05, 0.06, 0.07, 0.08, 0.09, and 0.1 mg/unit dose.

In some other embodiments, the concentration of the positive ion in the immune composition is 0.01 mg/ml to 0.1 mg/ml, for example, but not limited to, 0.01, 0.02, 0.03, 0.04, 0.05, 0.06, 0.07, 0.08, 0.09, and 0.1 mg/ml.

In some embodiments, the antigen derived from coronavirus is a virus (e.g. attenuated, inactivated virus, artificially assembled virus vector or virus particle).

In some specific embodiments, the antigen derived from coronavirus is an inactivated virus SARS-CoV-2, an attenuated virus SARS-CoV-2, or a virus SARS-CoV-2 that cannot be propagated in a subject. The ratio of the virus to the PIC is selected from the group consisting of 1 active unit/50 µg, 1 active unit/60 µg, 1 active unit/70 µg, 1 active unit/80 µg, 1 active unit/90 µg, 1 active unit/100 µg, 1 active unit/125 µg, 1 active unit/200 µg, 1 active unit/250 µg, 1 active unit/300 µg, 1 active unit/350 µg, 1 active unit/400 µg, 1 active unit/450 µg, 1 active unit/500 µg, 1 active unit/550 µg, 1 active unit/600 µg, 1 active unit/700 µg, 1 active unit/800 µg, 1 active unit/1000 µg, 1 active unit/1500 µg, 1 active unit/2000 µg, 1 active unit/2500 µg, 1 active unit/3000 µg, 1 active unit/4000 µg, 1 active unit/5000 µg, 1 active unit/6000 µg, 1 active unit/7000 µg, 1 active unit/8000 µg, 1 active unit/9000 µg, 1 active unit/10000 µg, and a range between any two values of the foregoing.

In some embodiments, when the antigen derived from coronavirus is a virus (e.g. attenuated, inactivated virus, artificially assembled virus vector or virus particle), the concentration of the antigen in the composition is 0.2 active units/unit dose to 100 active units/unit dose. More specifically, the concentration of the virus or antigen derived from the virus in the composition is selected from the group consisting of 0.2 active units/unit dose, 0.5 active units/unit dose, 1.0 active unit/unit dose, 1.5 active units/unit dose, 2.0 active units/unit dose, 2.5 active units/unit dose, 3.0 active units/unit dose, 3.5 active units/unit dose, 4.0 active units/unit dose, 5.0 active units/unit dose, 6.0 active units/unit dose, 7.0 active units/unit dose, 8.0 active units/unit dose, 9.0 active units/unit dose, 10.0 active units/unit dose, 15.0 active units/unit dose, 20.0 active units/unit dose, 30.0 active units/unit dose, 40.0 active units/unit dose, 50.0 active units/unit dose, 60.0 active units/unit dose, 70.0 active units/unit dose, 80.0 active units/unit dose, 90.0 active units/unit dose, 100.0 active units/unit dose, and a range between any two values of the foregoing. In a specific embodiment, the concentration of the virus or antigen derived from the virus in the composition is 0.5 active units/unit dose to 3.0 active units/unit dose; and preferably 1.0 active unit/unit dose to 2.5 active units/unit dose.

In some embodiments, when the antigen derived from coronavirus is a virus (e.g. attenuated, inactivated virus, artificially assembled virus vector or virus particle), the concentration of the antigen in the composition is 0.05 active units/ml to 40.0 active units/ml, preferably, 0.05 active units/ml, 0.1 active units/ml, 0.15 active units/ml, 0.2 active units/ml, 0.5 active units/ml, 1.0 active unit/ml, 2.0 active units/ml, 3.0 active units/ml, 4.0 active units/ml, 5.0 active units/ml, 10 active units/ml, 15 active units/ml, 20 active units/ml, 25 active units/ml, 30 active units/ml, 35 active units/ml, and 40 active units/ml.

In some embodiments, when the antigen derived from coronavirus is selected from the group consisting of an S protein of SARS-CoV-2 or an immunogenic fragment thereof, an M protein or an immunogenic fragment thereof, an N protein or an immunogenic fragment thereof, an E protein or an immunogenic fragment thereof, and a protein, a polypeptide, DNA and RNA designed according to the SARS-CoV-2 structure, the ratio of the antigen to the PIC is selected from the group consisting of 1 µg/10 µg , 1 µg/20 µg, 1 µg/30 µg, 1 µg/40 µg, 1 µg/50 µg, 1 µg/60 µg, 1 µg/70 µg, 1 µg/80 µg, 1 µg/90 µg, 1 µg/100 µg, 1 µg/125 µg, 1 µg/200 µg, 1 µg/250 µg, 1 µg/300 µg, 1 µg/350 µg, 1 µg/400 µg, 1 µg/450 µg, 1 µg/500 µg, 1 µg/550 µg, 1 µg/600 µg, 1 µg/700 µg, 1 µg/800 µg, 1 µg/1000 µg, 1 µg/1500 µg, 1 µg/2000 µg, 1 µg/2500 µg, 1 µg/3000 µg, 1 µg/4000 µg, 1 µg/5000 µg, 1 µg/6000 µg, 1 µg/7000 µg, 1 µg/8000 µg, 1 µg/9000 µg, 1 µg/ 10000 µg, and a range between any two values of the foregoing.

In some embodiments, when the antigen derived from coronavirus is selected from the group consisting of an S protein of SARS-CoV-2 or an immunogenic fragment thereof, an M protein or an immunogenic fragment thereof, an N protein or an immunogenic fragment thereof, an E protein or an immunogenic fragment thereof, and a protein, a polypeptide, DNA and RNA designed according to the SARS-CoV-2 structure, its concentration in the immune composition is 0.1 µg/unit dose to 1000.0 µg/unit dose, for example, but not limited to 0.5 µg/unit dose, 1.0 µg/unit dose, 2.0 µg/unit dose, 3.0 µg/unit dose, 4.0 µg/unit dose, 5.0 µg/unit dose, 6.0 µg/unit dose, 7.0 µg/unit dose, 8.0 µg/unit dose, 9.0 µg/unit dose, 10.0 µg/unit dose, 15.0 µg/unit dose, 20.0 µg/unit dose, 30.0 µg/unit dose, 40.0 µg/unit dose, 50.0 µg/unit dose, 60.0 µg/unit dose, 70.0 µg/unit dose, 80.0 µg/unit dose, 90.0 µg/unit dose, 100.0 µg/unit dose, 200.0 µg/unit dose, 300.0 µg/unit dose, 400.0 µg/unit dose, 500.0 µg/unit dose, 600.0 µg/unit dose, 700.0 µg/unit dose, 800.0 µg/unit dose, 900.0 µg/unit dose, 1000.0 µg/unit dose, and a range between any two values of the foregoing, or for example, 0.1 µg/ml to 1000.0 µg/ml; preferably 1 µg/ml, 2 µg/ml, 3 µg/ml, 5 µg/ml, 10 µg/ml, 20 µg/ml, 30 µg/ml, 40 µg/ml, 50 µg/ml, 100 µg/ml, 200 µg/ml, 500 µg/ml, and 1000 µg/ml.

In some embodiments, when the antigen derived from coronavirus is a SARS-CoV-2 spike protein, the concentration of the antigen in the immune composition is 2.5 µg/unit dose to 20 µg/unit dose.

In some embodiments, when the antigen derived from coronavirus is a SARS-CoV-2 spike protein multimer (e.g. a homotrimer), the concentration of the antigen in the immune composition is 2.5 µg/unit dose to 20 µg/unit dose.

In some embodiments, when the antigen derived from coronavirus is an immunogenic fragment of the SARS-CoV-2 spike protein, the concentration of the antigen in the immune composition is 2.5 µg/unit dose to 20 µg/unit dose.

In some embodiments, when the antigen derived from coronavirus is a SARS-CoV-2 spike protein RBD region, the concentration of the antigen in the immune composition is 2.5 µg/unit dose to 20 µg/unit dose.

In some embodiments, the unit dose described in the present disclosure is expressed in a form of volume, and is selected from the group consisting of 0.1 ml, 0.15 ml, 0.2 ml, 0.5 ml, 1.0 ml, 1.5 ml, 2.0 ml, 2.5 ml, 3.0 ml, 4.0 ml, 5.0 ml, 10.0 ml, 20.0 ml, 30.0 ml, 40.0 ml, 50.0 ml, 60.0 ml, 70.0 ml, 80.0 ml, 90.0 ml, 100.0 ml, 150 ml, 200 ml, 250.00 ml, and a range between any two values of the foregoing. The skilled person understands that too large or too small unit dose leads to inconveniences for clinical operation. Therefore, when the immune composition/vaccine of the present disclosure is injected and administered to a human subject, the unit dose is preferably in a range of 0.5 ml to 1.0 ml. When the immune composition/vaccine of the present disclosure is intranasally administered to a human subject, the unit dose is preferably in a range of 0.15 ml to 0.2 ml. When the immune composition/vaccine of the present disclosure is intravenously injected to a human subject, the unit dose is preferably in a range of 30.0 ml to 1000 ml. It should be understood herein that the unit dose, although expressed in volume, does not mean that the immune composition/vaccine of the present disclosure can only be in a liquid form. When the immune composition/vaccine of the present disclosure is prepared as a solid (dry powder or lyophilized powder), the volume of the unit dose refers to the volume after reformulation of the dry powder or lyophilized powder.

In some embodiments, the immune composition or vaccine of the present disclosure may contain gelatin, sucrose, white granulated sugar, lactose, maltose, trehalose, glucose, low molecular dextran, sorbitol, polysorbate 20, polysorbate 80, arginine hydrochloride, mannitol polyethylene glycol, human serum albumin, recombinant albumin, sodium caprylate, urea, aluminium hydroxide, phenol red, aluminium phosphate, squalene, saponin, oligonucleotide, magnesium chloride, potassium chloride, sodium chloride, sodium thiosulfate, potassium dihydrogen phosphate, ascorbic acid, chloroform, phenol, and thimerosal.

In some embodiments, the immune composition or vaccine of the present disclosure may further contain a physiologically acceptable buffer solution, selected from the group consisting of acetate, Tris, bicarbonate, carbonate, and a phosphate buffer solution. The pH values of the buffer solution suitable for the composition of the present disclosure are selected from the group consisting of: 6.50, 6.60, 6.70, 6.80, 6.90, 7.00, 7.05, 7.1, 7.15, 7.2, 7.25, 7.30, 7.35, 7.40, 7.45, 7.50, 7.55, 7.60, 7.65, 7.70, 7.75, 7.80, 7.85, 7.90, 7.95, 8.00, and a range between any two values of the foregoing. In a specific embodiment, the buffer solution is PBS, and the pH value is within a range of 7.0 to 8.0.

In some embodiments, the concentration of the buffer solution in the composition is 5 mM to 50 mM, specifically, 5 mM to 20 mM. Examples that may be mentioned are 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50 mM.

After the buffer solution is prepared into the composition or vaccine of the present disclosure, it is not excluded that its pH will change with the addition of other ingredients. In view of the buffer capacity of the buffer solution, in some embodiments, the pH value of the immune composition or vaccine of the present disclosure is controlled in a suitable range, for example: 6.0, 6.1, 6.2, 6.3, 6.4, 6.5, 6.6, 6.7, 6.8, 6.9, 7.0, 7.05, 7.1, 7.15, 7.2, 7.25, 7.3, 7.35, 7.4, 7.45, 7.5, 7.55, 7.6, 7.65, 7.7, 7.75, 7.8, 7.85, 7.9, 7.95, 8.0, 8.1, 8.2, 8.3, 8.4, 8.5, 8.6, 8.7, 8.8, 8.9, 9.0, and a range between any two values of the foregoing. In a specific embodiment, the pH value of the composition is within a range of 7.0 to 8.0.

The immune composition or vaccine of the present disclosure may be prepared as dry powder, liquid solution (e.g. injectable solution, aqueous or saline solution, or suspension, ointment, droplet, emulsion, gel, syrup or slurry, aerosol, spray, naristillae), tablet, coated tablet, microcapsule, suppository, pill, granule, sugar-coated lozenge, and capsule. The preparation method is generally described in the fourth edition of Vaccine by Stanley A Plotkin et al., W. B. Saunders Company 2003. Preferably, the immune composition or vaccine of the present disclosure is prepared as an injectable solution.

According to another aspect of the present disclosure, an immune composition is provided in a form of dry powder or lyophilized powder corresponding to the above immune composition. The skilled person could appreciate that when the immune composition is in the form of dry powder or lyophilized powder, it is different from the immune composition in a liquid form only in the water content. As an example, when the immune composition in a dry powder or lyophilized powder form contains 2 mg/ml PIC, the skilled person knows that this concentration refers to the concentration of PIC after reformulation of the immune composition into the liquid form.

In some embodiments, an immune composition is provided, which contains:
an antigen derived from coronavirus,
0.5 mg/ml to 2 mg/ml PIC,
400 units/ml to 1200 units/ml kanamycin,
0.01 mg/ml to 0.1 mg/ml calcium chloride,
100 mM to 200 mM sodium chloride, and
5 mM to 20 mM phosphate buffer solution.

In some embodiments, an immune composition is provided, which contains:
an antigen derived from coronavirus,
0.5 mg/ml to 2 mg/ml PIC,
400 units/ml to 1200 units/ml kanamycin,
0.01 mg/ml to 0.1 mg/ml calcium chloride,
100 mM to 200 mM sodium chloride,
5 mM to 20 mM phosphate buffer solution,
optionally, 100 mM to 200 mM arginine hydrochloride,
optionally, 0.005% w/v to 0.05% w/v polysorbate 80, and
optionally, 0.1% w/w to 1.0% w/w aluminium phosphate.

### Method and use

According to another aspect of the present disclosure, there is provided use of a combination of the above PIC, antibiotic (or polyamino compound), and positive ion in preparation of a medicament or vaccine for prevention or treatment of coronavirus infection.

According to yet another aspect of the present disclosure, there is provided use of a polynucleotide adjuvant composition disclosed in CN103405762A in preparation of a medicament or vaccine for prevention or treatment of coronavirus infection.

According to another aspect of the present disclosure, there is provided use of a combination of PIC, antibiotic (or polyamino compound), positive ion, and virus in preparation of a medicament or vaccine for prevention or treatment of coronavirus infection.

According to another aspect of the present disclosure, there is provided use of an immune composition or vaccine of the present disclosure in prevention or treatment of coronavirus infection.

More specifically, there is provided use of an immune composition or vaccine of the present disclosure in preparation of a medicament for prevention or treatment of coronavirus infection.

According to another aspect of the present disclosure, a method of stimulating an immunoreaction in a host animal is further involved, wherein the method includes administering to the animal the immune composition herein in an amount effective to induce immunoreaction. The host animal is preferably a mammal, more preferably a monkey, and still more preferably a human. The present disclosure further relates to a method for immunizing a host animal against a virus, wherein the method includes administering to the animal the immune composition herein in an amount effective to induce protective reaction. The host animal is preferably a mammal, more preferably a monkey, and still more preferably a human.

The "immunoreaction" to the antigen or composition is a reaction generated in a subject's humoral and/or cellular immunoreaction to molecules existing in a composition of interest. The "humoral immunoreaction" refers to an antibody molecule-mediated immunoreaction, while the "cellular immunoreaction" is an immunoreaction mediated by T-lymphocytes and/or other leukocytes.

In some specific embodiments, the immune composition or vaccine of the present disclosure is capable of achieving any one selected from the group consisting of upregulating the expression of costimulatory molecules (e.g. CD80+, CD86+, CD40+) on dendritic cells, activating dendritic cells, increasing the expression of cytokines (e.g. TNF-α, IFN-γ, KC, IFN-β) in the lung, increasing the titer of IgG antibodies (e.g. IgG1, IgG2a, IgG3), increasing the ratio of IL-2 positive cells, increasing the ratio of IFN-γ positive cells, and decreasing the virus load in the lungs or a combination thereof.

According to another aspect of the present disclosure, there is provided a method for preventing or treating coronavirus infection, which includes administering to a subject a prophylactically or therapeutically effective amount of the composition or vaccine of the present disclosure.

In some embodiments, the administration of the composition is systematic (i.e., systemic) or local.

In some embodiments, the immune composition or vaccine of the present disclosure is administered by parenteral (e.g. intramuscular, intraperitoneal, intravenous, subcutaneous, and intradermal) injection.

In other embodiments, the immune composition or vaccine of the present disclosure is intradermally transmitted in a manner other than injection (e.g. in a manner that does not destroy epithelial cell barriers by a mechanical device).

In some other embodiments, the immune composition is administered through rectal, vaginal, nasal, oral, sublingual, respiratory tract, ocular, or transdermal routes.

In some embodiments, the immune composition is administered intramuscularly.

In some embodiments, the immune composition is administered by nasal drip.

In some embodiments, for inhalation administration, the composition/vaccine of the present disclosure may be administered by an insufflator, an atomizer, a pressure package or by introducing a gas spray. The pressure package may carry a suitable propellant (e.g. dichlorodifluoromethane, trichlorofluoromethane, dichlorotetrafluoroethane, and carbon dioxide). In the case of using pressurized aerosol, the dose unit may be determined by providing a gas valve.

Optionally, for inhalation or insufflation administration, the composition/vaccine of the present disclosure is a composition in the form of dry powder, for example, a powdered mixture of a modifier and a suitable dry powder matrix such as lactose or starch. The powdered mixture can be contained in capsules and cartridges in a form of unit dose, or for example, in gelatin package, from which containers the medicine powder can be administered by means of an inhaler or an insufflator. For nasal delivery, the antibody can be administered by liquid spray or naristillae, such as by a plastic sprayer.

In some embodiments, the immune composition or vaccine of the present disclosure is administered to a subject at a frequency selected from the group consisting of once a month, twice a month, 3 times a month, 4 times a month, 5 times a month, 6 times a month, 7 times a month, 8 times a month, once a week, twice a week, 3 times a week, 4 times a week, 5 times a week, 6 times a week, once every three days, twice every three days, 3 times every three days, once every two days, twice every two days, once a day, and twice a day.

In some embodiments, the method for preventing or treating includes administering the immune composition of the present disclosure containing a SARS-CoV-2 antigen, and it is also feasible to administer a composition of nucleic acid encoding the SARS-CoV-2 antigen. Those skilled in the art are familiar with the concepts, applications, and effectiveness of nucleic acid vaccines, nucleic acid vaccine technologies, and technologies based on protein and polypeptide. The nucleic acid-based technology enables direct application of naked or encapsulated nucleic acid encoding a spike polypeptide to tissues and cells. This technology is based on the fact that these nucleic acids can be ingested and expressed by cells of a subject organism to produce immunogenic determinants. Such protein-based vaccines can induce neutralizing, protective and antibody-dependent immune response through single or multiple injections of the immune composition of the present disclosure (e.g. SARS-CoV-2-containing spike protein).

According to another aspect of the present disclosure, there is provided a kit for carrying out the above method for preventing or treating, including at least one container containing the immune composition or vaccine of the present disclosure. Ingredients and/or amounts of the composition in different containers may be the same or different.

In some embodiments, the immune composition or vaccine of the present disclosure is formulated in a sterile liquid, and is contained in a sterile container (e.g. a tube, a bottle, an ampoule, and a syringe). In some other embodiments, the immune composition or vaccine of the present disclosure is contained in a container in a form of dry powder or lyophilized powder. Prior to use, the immune composition or vaccine is formulated in a liquid form.

In some embodiments, the kit of the present disclosure further includes one selected from the group consisting of a needle, water for injection, and instructions for use, or a combination thereof.

### BRIEF DESCRIPTION OF DRAWINGS

FIG. 1 shows a pharmaceutical composition of the present disclosure activating body DC to upregulate costimulatory molecule production.
FIG. 2A to FIG. 2B show that the concentration of cytokines in the lungs can be effectively increased after administration of the pharmaceutical composition of the present disclosure to mice by nasal drip.
FIG. 3: Titers of IgG subtype antibodies.
FIG. 4: Titers of IgG antibodies against novel coronavirus S protein in rabbit serum.
FIG. 5: Neutralizing antibodies induced by different pharmaceutical compositions have different titers.
FIG. 6A to FIG. 6D: Proportion of cells expressing IL-2, IFN-γ.
FIG. 7: Virus load in lungs after challenge of mice.
FIG. 8: Virus load in lungs after challenge of mice.

### DETAILED DESCRIPTION OF EMBODIMENTS

"Antigen" refers to any substance (e.g. protein, peptide, cancer cell, glycoprotein, glycolipid, live virus, killed virus, and DNA) that can be recognized by a host's immune system and induce an immune response when it invades the host. The antigen can be provided in a purified or unpurified form. The antigen in the present disclosure includes protein of pathogen, recombinant protein, peptide, polysaccharide, glycoprotein, glycolipid and polynucleotide, live virus, recombinant virus, attenuated or inactivated virus.

Immunogenic fragment refers to a portion of the foregoing "antigen" (e.g. when the antigen is a protein or polypeptide, the immunogenic fragment is a non-full-length peptide fragment of the protein or polypeptide), which still retains (in whole or in part) the ability to be recognized by the host's immune system and induce the immune response.

Inactivation refers to removing the pathogenic ability and propagation ability of the virus, but still retain the ability to stimulate the human body to produce the immune response. The methods for inactivating viruses are well known in the art. Any commonly used method can be used to inactivate viruses, and can be selected as appropriate according to virus types. These methods for inactivating viruses include, but are not limited to, using a photoreactive compound, an oxidant, radiation (e.g. UV rays; γ-rays), a combination of riboflavin and UV rays, solvent-detergent treatment (e.g. treatment with organic solvent tri-N-butyl-phosphate and Tween 80 detergent), polyethylene glycol treatment, pasteurization (heat treatment), low pH treatment, enzyme treatment (pepsin or trypsin), methylene blue-light treatment, treatment with dimethylmethylene blue and visible light, and treatment with psoralen derivative S-59 and UVA irradiation.

Attenuation: Attenuated viruses are still alive, while the viruses lose pathogenicity during preparation, but still retain the propagation capacity and the ability to stimulate the human body to produce an immune response.

Herein, the term spike protein (Spike or S protein) covers variants thereof. The variant of spike protein refers to a polypeptide that is substantially homologous to natural spike polypeptide, but has an amino acid sequence different from the natural spike protein due to the deletion, insertion or substitution of one or more amino acids. The variant may contain a sequence of conservative substitution, wherein the conservative substitution refers to substitution of a given amino acid residue by a residue having similar biochemical properties. Such conservative substitution is known, and examples of conservative substitution include substitution of aliphatic residues with each other, such as substitution of Ile, Val, Leu, or Ala with each other; or substitution of polar residues with each other, for example, between Lys and Arg; between Glu and Asp; and between Gln and Asn. The present disclosure also includes naturally occurring spike polypeptide variants. Examples of such variants are proteins resulting from alternative mRNA splicing events or protease cleavage of the spike polypeptide. Variations that may be attributable to proteolysis include, for example, variations having differences in terminal upon expression in different types of host cells, due to proteolytic removal of one or more terminal amino acids from the spike polypeptide.

When the SARS-CoV-2 antigen of the present disclosure is a protein or polypeptide, it is possible to prepare it into a form of multimer (homomultimer or heteromultimer), for example, but not limited to, dimer, trimer, tetramer, and pentamer, thereby providing more antigen contact opportunities. In a specific example, the skilled person fuses a segment of fold at the N- or C-terminus of the SARS-CoV-2 antigen, this segment of fold facilitates the antigen in forming the multimer form. Such fold is well known in the art and does not affect the correct folding of the antigen in the present disclosure or affect the immunogenicity. As an example, folds that can be used for trimerization allow for the formation of trimeric structures when attached (also referred to as fused) to proteins or peptides. In the present disclosure, any known trimerization fold/domain can be used. Examples of trimerization folds/domains include, but are not limited to: HIV-1gp41 trimerization domain, SIV gp41 trimerization domain, Ebola virus gp-2 trimerization domain, HTLV-1gp-21 trimerization domain, T4 fibritin trimerization domain, yeast heat shock transcription factor trimerization domain, and human collagen trimerization domain. For another example, in CN111560074A, a twenty-four-polymer nano antigen can be formed by HP_Ferritin self-assembly function; and in CN111607605A, a pentamer of fusion protein is obtained by means of the characteristic that LTB26 can be self-assembled to form a pentamer.

The term "unit dose" as used in the present disclosure refers to a unit that is physically isolated, and suitable as a unit dose used for a subject individual. Each unit contains a predetermined amount of the composition of the present disclosure, wherein the predetermined amount is an amount sufficient to produce the desired effect together with a pharmaceutically/physiologically acceptable diluent, carrying agent or carrier.

The term "effective amount" of a composition herein refers to an amount of the composition that is non-toxic but sufficient to provide a desired reaction (e.g. an immunoreaction) and produce a corresponding preventive/therapeutic effect. The exact amount required varies from subject to subject, depending on their species, ages, overall conditions of the subjects, the severity of the condition to be treated, and particular macromolecules of interest, the manner of administration, etc. The suitable "effective amount" in a particular example can be determined by one of ordinary skill in the art using conventional experimental methods, for example, determining an effective amount of a human subject through animal studies.

The term "treatment" as used in the present description refers to obtaining a desired pharmacological and/or physiological effect. The effect may be preventive from the perspective of completely and/or partially preventing diseases or symptoms thereof, and/or the effect may be medical from the perspective of completely and/or partially stabilizing or curing diseases and/or negative effects caused by the diseases.

In the present description, when a numerical range is described, the expressions "...to...", "within a range" or "between ranges" used include endpoint values.

In the context of the present description, pharmaceutical composition, medicine (drug), immune composition, and vaccine may be used interchangeably.

### Examples

Unless otherwise specified, the operation for test animals of the present disclosure follows *Regulations for the Administration of Laboratory Animals, Guidelines for the Humane Treatment of Laboratory Animals,* and National Standard GB/14925.

### Example 1: Preparation method of pharmaceutical composition 1 of the present disclosure

1. Antigen: recombinant novel coronavirus S-trimer protein.
2. Preparation of the composition of the present disclosure according to the following ingredients:
   the composition 1 (with a unit dose of 1 ml) of the present disclosure contains:
   recombinant novel coronavirus S-trimer protein (2.5 µg, 5 µg, 6 µg, 10 µg, and 20 µg),
   PIC (1 mg/ml),
   kanamycin (800 units/ml), and
   calcium chloride (0.0472 mg/ml).

The above composition was formulated in a physiologically acceptable buffer solution (sodium chloride 150 mM, disodium hydrogen phosphate 0.0043 M, and sodium dihydrogen phosphate 0.0017 M) under sterile conditions.

**Table 1**

| **Ingredients** | **Dosage (1 ml)** | **Concentration** |
|---|---|---|
| recombinant novel coronavirus | 2.5 µg, | 2.5 µg/ml, |
| S-trimer protein | 5 µg, | 5 µg/ml, |
| | 6 µg, | 6 µg/ml, |
| | 10 µg, | 10 µg/ml, |
| | 20 µg, | 20 µg/ml, |
| PIC | 1 mg | 1 mg/ml |
| kanamycin | 800 units | 800 units/ml |
| calcium chloride | 0.0472 mg | 0.0472 mg/ml |
| sodium chloride | 8.77 mg | 150 mM |
| disodium hydrogen phosphate | 0.61 mg | 0.0043 M |
| sodium dihydrogen phosphate | 0.204 mg | 0.0017 M |

### Example 2: Preparation method of pharmaceutical composition 2 of the present disclosure

1. Antigen: recombinant novel coronavirus S-trimer protein.
2. Preparation of the composition of the present disclosure according to the following ingredients:
   the composition 2 (with a unit dose of 1 ml) of the present disclosure contains:
   recombinant novel coronavirus S-trimer protein (2.5 µg, 5 µg, 10 µg, and 20 µg),
   PIC (1 mg/ml),
   kanamycin (800 units/ml),
   calcium chloride (0.0472 mg/ml),
   arginine hydrochloride (140 mM), and
   polysorbate 80 (0.01% w/v).

The above composition was formulated in a physiologically acceptable buffer solution (sodium chloride 150 mM, disodium hydrogen phosphate 0.0043 M, and sodium dihydrogen phosphate 0.0017 M) under sterile conditions.

**Table 2**

| **Ingredients** | **Dosage (1 ml)** | **Concentration** |
|---|---|---|
| recombinant novel | 2.5 µg, | 2.5 µg/ml, |
| coronavirus S-trimer protein | 5 µg, | 5 µg/ml, |
| | 10 µg, | 10 µg/ml, |
| | 20 µg, | 20 µg/ml, |
| PIC | 1 mg | 1 mg/ml |
| kanamycin | 800 units | 800 units/ml |
| calcium chloride | 0.0472 mg | 0.0472 mg/ml |
| arginine hydrochloride | 29.492 mg | 140 mM |
| polysorbate 80 | 0.1 mg | 0.01% w/v |
| sodium chloride | 8.77 mg | 150 mM |
| disodium hydrogen phosphate | 0.61 mg | 0.0043 M |
| sodium dihydrogen phosphate | 0.204 mg | 0.0017 M |

### Example 3: Preparation method of pharmaceutical composition 3 of the present disclosure

1. Antigen: recombinant novel coronavirus S1 protein.
2. Preparation of the composition of the present disclosure according to the following ingredients:
   the composition 3 (with a unit dose of 1 ml) of the present disclosure contains:
   recombinant novel coronavirus S1 protein (2.5 µg, 5 µg, 6 µg, 10 µg, and 20 µg),
   PIC (1 mg/ml),
   kanamycin (800 units/ml), and
   calcium chloride (0.0472 mg/ml).

The above composition was formulated in a physiologically acceptable buffer solution (sodium chloride 150 mM, disodium hydrogen phosphate 0.0043 M, and sodium dihydrogen phosphate 0.0017 M) under sterile conditions.

**Table 3**

| **Ingredients** | **Dosage (1 ml)** | **Concentration** |
|---|---|---|
| recombinant novel | 2.5 µg, | 2.5 µg/ml, |
| coronavirus S1 protein | 5 µg, | 5 µg/ml, |
| | 6 µg, | 6 µg/ml, |
| | 10 µg, | 10 µg/ml, |
| | 20 µg, | 20 µg/ml, |
| PIC | 1 mg | 1 mg/ml |
| kanamycin | 800 units | 800 units/ml |
| calcium chloride | 0.0472 mg | 0.0472 mg/ml |
| sodium chloride | 8.77 mg | 150 mM |
| disodium hydrogen phosphate | 0.61 mg | 0.0043 M |
| sodium dihydrogen phosphate | 0.204 mg | 0.0017 M |

### Example 4: Preparation method of pharmaceutical composition 4 of the present disclosure

1. Antigen: recombinant novel coronavirus RBD protein (i.e., spike protein RBD region of SARS-CoV-2).
2. Preparation of the composition of the present disclosure according to the following ingredients:
   the composition 4 (with a unit dose of 1 ml) of the present disclosure contains:
   recombinant novel coronavirus RBD protein (2.5 µg, 5 µg, 6 µg, 10 µg, and 20 µg),
   PIC (1 mg/ml),
   kanamycin (800 units/ml), and
   calcium chloride (0.0472 mg/ml).

The above composition was formulated in a physiologically acceptable buffer solution (sodium chloride 150 mM, disodium hydrogen phosphate 0.0043 M, and sodium dihydrogen phosphate 0.0017 M) under sterile conditions.

**Table 4**

| **Ingredients** | **Dosage (1 ml)** | **Concentration** |
|---|---|---|
| Recombinant novel | 2.5 µg, | 2.5 µg/ml, |
| coronavirus RBD protein | 5 µg, | 5 µg/ml, |
| | 6 µg, | 6 µg/ml, |
| | 10 µg, | 10 µg/ml, |
| | 20 µg, | 20 µg/ml, |
| PIC | 1 mg | 1 mg/ml |
| kanamycin | 800 units | 800 units/ml |
| calcium chloride | 0.0472 mg | 0.0472 mg/ml |
| sodium chloride | 8.77 mg | 150 mM |
| disodium hydrogen phosphate | 0.61 mg | 0.0043 M |
| sodium dihydrogen phosphate | 0.204 mg | 0.0017 M |

### Example 5: Preparation method of pharmaceutical composition 5 of the present disclosure

Composition 5 (with a unit dose of 1 ml) of the present disclosure contains:
PIC (1 mg/ml),
kanamycin (800 units/ml), and
calcium chloride (0.0472 mg/ml).

The above composition was formulated in a physiologically acceptable buffer solution (sodium chloride 150 mM, disodium hydrogen phosphate 0.0043 M, and sodium dihydrogen phosphate 0.0017 M) under sterile conditions.

**Table 5**

| **Ingredients** | **Dosage (1 ml)** | **Concentration** |
|---|---|---|
| PIC | 1 mg | 1 mg/ml |
| kanamycin | 800 units | 800 units/ml |
| calcium chloride | 0.0472 mg | 0.0472 mg/ml |
| sodium chloride | 8.77 mg | 150 mM |
| disodium hydrogen phosphate | 0.61 mg | 0.0043 M |
| sodium dihydrogen phosphate | 0.204 mg | 0.0017 M |

### Example 6: Preparation method of pharmaceutical composition 6 of the present disclosure

1. Antigen: inactivated and purified novel coronavirus.
2. Preparing the composition 6 (with a unit dose of 1 ml) of the present disclosure according to the following ingredients, containing:
   inactivated and purified novel coronavirus (600 active units),
   PIC (1 mg/ml),
   kanamycin (800 units/ml),
   calcium chloride (0.0472 mg/ml), and
   aluminium phosphate (with an aluminium content of 0.5%w/w).

The above composition was formulated in a physiologically acceptable buffer solution (sodium chloride 150 mM, disodium hydrogen phosphate 0.0043 M, and sodium dihydrogen phosphate 0.0017 M) under sterile conditions.

**Table 6**

| **Ingredients** | **Dosage (1 ml)** | **Concentration** |
|---|---|---|
| inactivated and purified novel coronavirus | 600 active units | 600 active units/ml |
| PIC | 1 mg | 1 mg/ml |
| kanamycin | 800 units | 800 units/ml |
| calcium chloride | 0.0472 mg | 0.0472 mg/ml |
| aluminium phosphate | 0.005 ml | 0.5% w/w |
| sodium chloride | 8.77 mg | 150 mM |
| disodium hydrogen phosphate | 0.61 mg | 0.0043 M |
| sodium dihydrogen phosphate | 0.204 mg | 0.0017 M |

### Test Example 1: The pharmaceutical composition 5 of the present disclosure activates dendritic cells of the body to upregulate costimulatory molecules

Method: C57BL/6 mouse myeloid-derived dendritic cells (BMDC) were *in vitro* co-incubated with the pharmaceutical composition 5 of the present disclosure for 18 hours. The proportions and mean fluorescence intensities of CD80+, CD86+, and CD40+ cells were detected with flow cytometry.

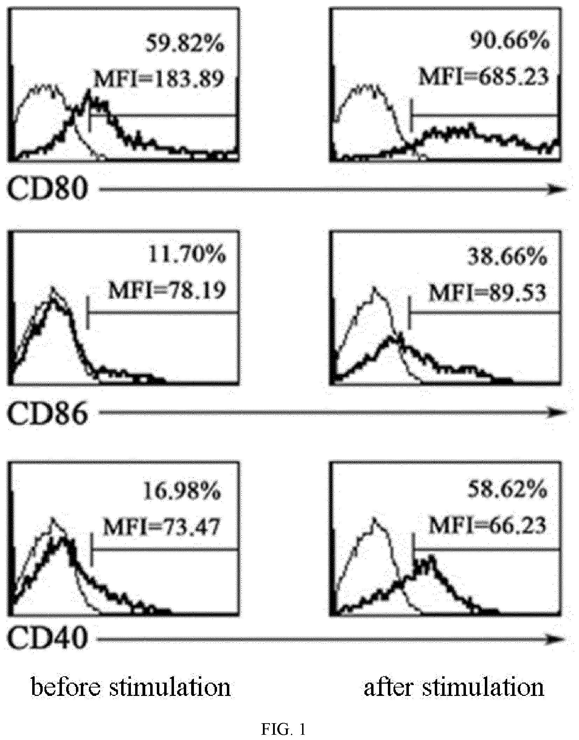

Result: As shown in FIG. 1, after the pharmaceutical composition 5 of the present disclosure interacted with the mouse BMDC, the proportion of CD80+ cells was increased from 59.82% to 90.66%, the proportion of CD86+ cells was increased from 11.70% to 38.66%, and the proportion of CD40+ cells was increased from 16.98% to 58.62%, significantly upregulating the production of costimulatory molecules.

Conclusion: The present experiment proves that the pharmaceutical composition 5 of the present disclosure can significantly induce dendritic cells of the body to upregulate costimulatory molecules, and promote activation of the dendritic cells.

### Test Example 2: After nasal delivery, the pharmaceutical composition 5 of the present disclosure can effectively increase the concentration of cytokines in the lungs.

Method: The mice were intranasally administered with PBS or the pharmaceutical composition 5 of the present disclosure, respectively, and sacrificed at specified time. Lung tissues were collected, and the TNF-α, IFN-γ, and KC of the sample were repeatedly tested by a Bio-plex protein array system; and IFN-β was detected with an ELISA kit.

**Table 7. Laboratory Animals Grouping**

| Samples | Administration Dosage | Administration Mode |
|---|---|---|
| PBS | 50 µl per mouse | *i.n.* |
| pharmaceutical composition 5 of the present disclosure | 50 µl per mouse | *i.n.* |

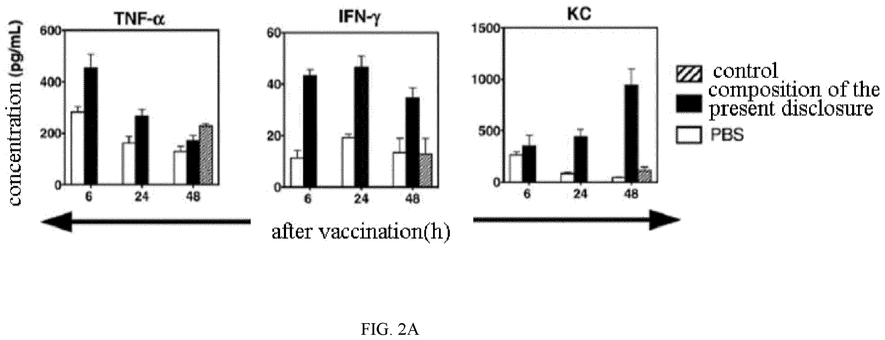

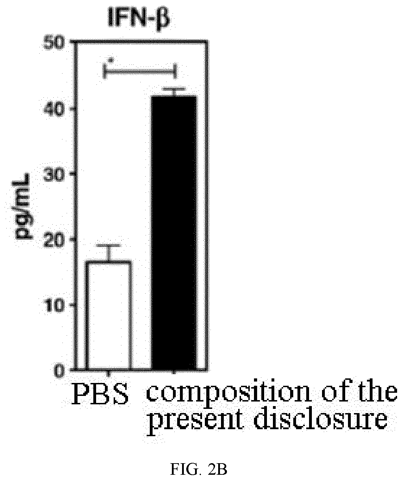

Result: See FIG. 2A to FIG. 2B.

Conclusion: After intranasally administering the mice with the pharmaceutical composition 5 of the present disclosure, the cytokines TNF-α, IFN-γ, KC, and IFN-β in the mouse lungs were significantly increased, indicating that nasally administering the mice can effectively activate the intrinsic immunity of the respiratory tract, causing the immune cells to produce cytokines and chemokines. These cytokines and chemokines are associated with inhibition of the respiratory virus replication of mice.

### Test Example 3: The pharmaceutical composition 1 of the present disclosure can effectively induce the mice to produce IgG antibodies (IgG1, IgG2a, IgG3) against the novel coronavirus S1 protein

Method: The pharmaceutical composition 1 of the present disclosure was used to immunize the mice. The blood was taken, and the serum was isolated, Titers of IgG antibody subtypes against the novel coronavirus S 1 protein in mouse serum was detected by the ELISA method.

**Table 8. Laboratory Animals Grouping**

| Sample | Administration Dosage | Administration Mode | Immunization Time | Number |
|---|---|---|---|---|
| pharmaceutical composition 1 of the present disclosure (antigen of 10 µg) | 0.1 ml per mouse | *i.m.* | immunizing once on days 0, 7, and 14 | 8 |

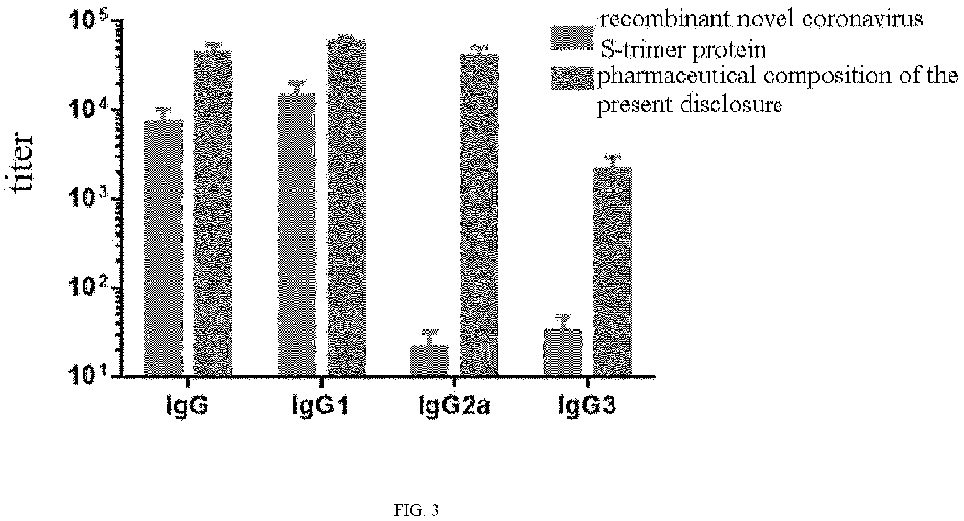

Conclusion: The pharmaceutical composition of the present disclosure can significantly increase the titers of IgG subtype antibodies against the novel coronavirus S1 protein in serum (FIG. 3).

### Test Example 4: The pharmaceutical composition 1 of the present disclosure can effectively induce rabbits to produce IgG antibodies against the novel coronavirus S protein

The rabbits were immunized with the pharmaceutical composition 1 of the present disclosure. The blood was taken, and the serum was isolated. Titers of the IgG antibodies against the novel coronavirus S protein in the rabbit serum was detected by the ELISA method.

**Table 9. Laboratory Animals Grouping**

| Sample | Administration Dosage | Administration Mode | Immunization Time | Number |
|---|---|---|---|---|
| pharmaceutical composition 1 (antigen of 5 µg) of the present disclosure | 1 ml per rabbit | *i.m.* | immunizing once on days 0 and 7 | 5 |

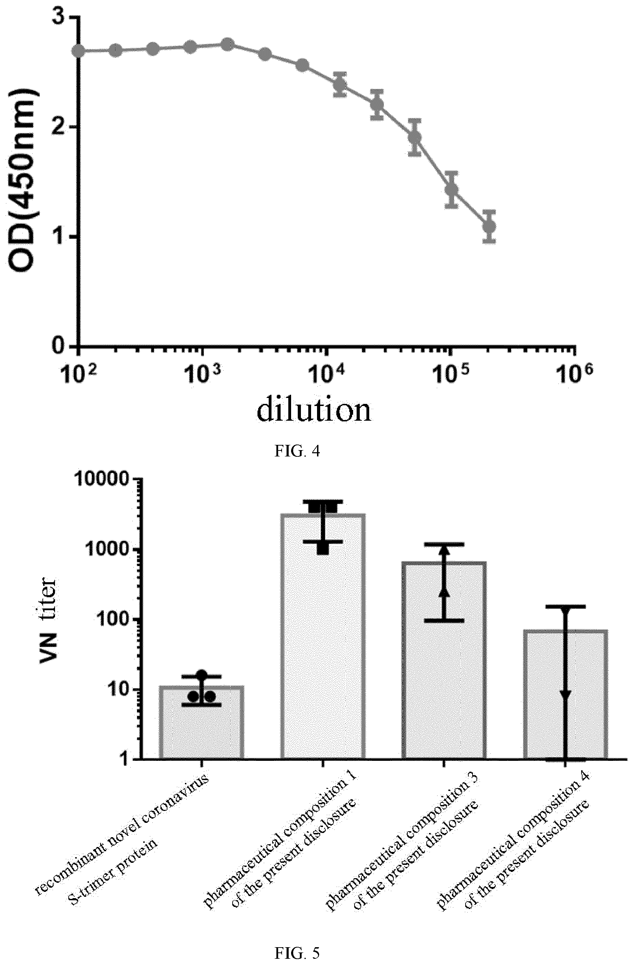

Conclusion: The pharmaceutical composition of the present disclosure can significantly increase the titers of IgG antibodies in the rabbit serum against the novel coronavirus S protein (FIG. 4).

### Test Example 5: The pharmaceutical compositions 1 and 3-4 of the present disclosure can effectively induce rabbits to produce neutralizing antibodies against the novel coronavirus

Method: The pharmaceutical compositions 1 and 3-4 of the present disclosure were used respectively to immunize the rabbits. The blood was taken, and the serum was isolated. Titers of neutralizing antibodies in the rabbit serum was detected through pseudovirus neutralizing test.

**Table 10. Laboratory Animals Grouping**

| Samples | Administration Dosage | Administration Mode | Immunization Time | Number |
|---|---|---|---|---|
| pharmaceutical composition 1 (antigen of 6 µg) of the present disclosure | 1 ml per rabbit | *i.m.* | immunizing once on days 0, 7, and 14 | 3 |
| pharmaceutical composition 3 (antigen of 6 µg) of the present disclosure | 1 ml per rabbit | *i.m.* | immunizing once on days 0, 7, and 14 | 3 |
| pharmaceutical composition 4 (antigen of 6 µg) of the present disclosure | 1 ml per rabbit | *i.m.* | immunizing once on days 0, 7, and 14 | 3 |

Conclusion: The pharmaceutical compositions 1 and 3-4 of the present disclosure can significantly increase the titers of neutralizing antibodies in rabbit serum, and titers of neutralizing antibodies induced and produced by different pharmaceutical compositions are different (FIG. 5).

### Test Example 6: The pharmaceutical composition 2 of the present disclosure effectively induces the mice to produce novel coronavirus-specific cellular immunoreaction

Method: The pharmaceutical composition 2 of the present disclosure was used to immunize the mice. Spleen was taken, and a spleen cell suspension was isolated. Antigen-specific cellular immunoreaction was detected by the Elispot method.

**Table 11. Laboratory Animals Grouping**

| Sample | Administration Dosage | Administration Mode | Immunization Time | Number |
|---|---|---|---|---|
| pharmaceutical composition 2 (antigen: low dosage 2.5 µg, medium dosage: 5 µg, and high dosage: 10 µg) of the present disclosure | 0.1 ml per mouse | *i.m.* | immunizing once on days 0, 7, and 14 | 8 |

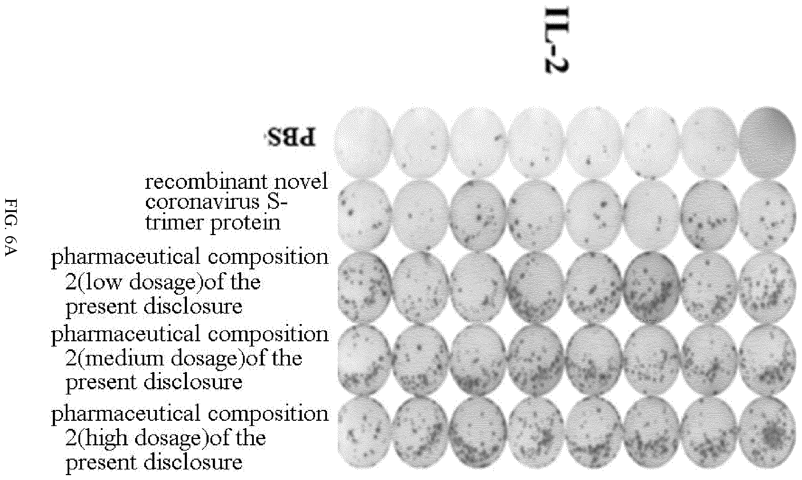

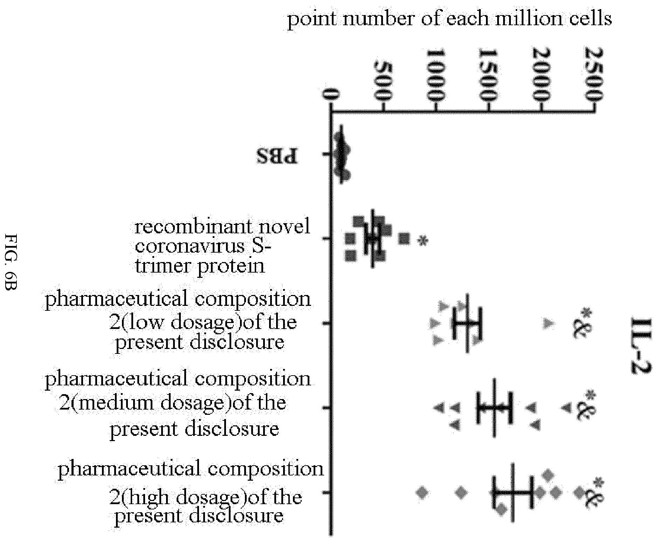

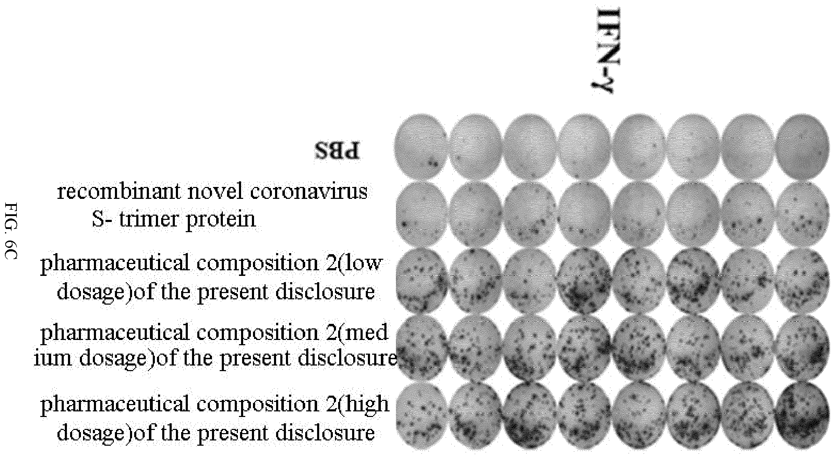

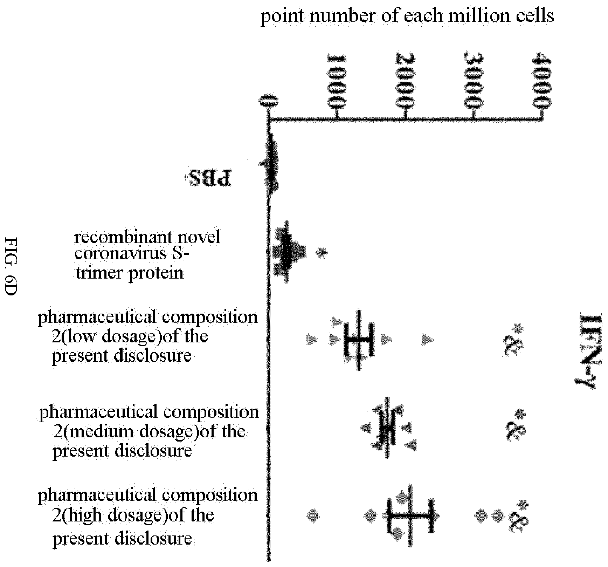

Conclusion: The pharmaceutical composition 2 of the present disclosure can significantly improve the mouse cellular immunoreaction, and significantly increase the proportions of cells expressing IL-2 and IFN-γ (see FIG. 6A to FIG. 6D for the results).

### Test Example 7: The pharmaceutical composition 1 of the present disclosure effectively reduces the novel coronavirus load in hACE2 mice

Method: The hACE2 transgenic mice (6-week old, 18-26 g, SARS-CoV-2 infected hACE2 transgenic mouse models) were immunized with the pharmaceutical composition 1 of the present disclosure, and were subjected to challenge by nasal drip with SARS-CoV-2 14 days after primary immunization, and 5 days after the challenge, lung tissues were taken to detect the lung virus load.

**Table 12. Laboratory Animals Grouping**

| Samples | Administration Dosage | Administration Mode | Immunization Time | Challenge Time | Challenge Dosage and Volume | Number |
|---|---|---|---|---|---|---|
| pharmaceutical composition 1 (antigen of 5 µg) of the present disclosure | 200 µl per mouse | *i.m.* | immunizing once on days 0 and 7 | challenge 14 days after the primary immunization | 10⁵ TCID₅₀ per mouse 50 µl | 6 |

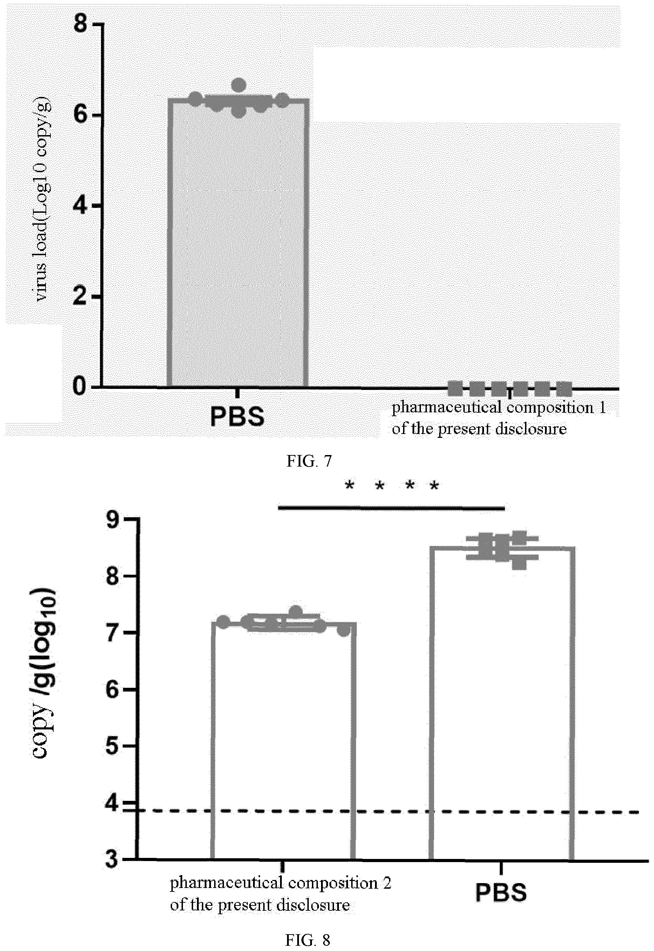

Conclusion: The pharmaceutical composition 1 of the present disclosure can significantly reduce the lung virus load of the mice after challenge, relieve the pulmonary inflammation to some extent, and have a good preventive effect (see FIG. 7 for the results).

### Test Example 8: The pharmaceutical composition 2 of the present disclosure effectively reduces the novel coronavirus load in hACE2 mice

Method: The hACE2 mice were subjected to intranasal challenge with SARS-CoV-2, and simultaneously administered with the pharmaceutical composition 2 of the present disclosure. The lung tissues were taken 5 days after the challenge to detect the virus load in lungs.

**Table 13. Laboratory Animals Grouping**

| Sample | Administration Dosage | Administration Mode | Immunization Time | Challenge Time | Challenge Dosage and Volume | Number |
|---|---|---|---|---|---|---|
| pharmaceutical composition 2 (antigen of 10 µg) of the present disclosure | 200 µl per mouse | *i.m.* | administering continuously 5 days before challenge, and stopping immunization on the 4th day after challenge | the 6th day after the first time of immunization | 0.5^{∗}10⁶ TCID₅₀ per mouse 50 µl | 6 |

Conclusion: The pharmaceutical composition 2 of the present disclosure can significantly reduce the lung virus load of the mice after challenge, and have a good therapeutic effect (see FIG. 8 for the results).

### Test Example 9: The pharmaceutical composition 2 of the present disclosure can effectively induce machin to produce IgG antibodies against the novel coronavirus S protein

Method: The machins were immunized with the pharmaceutical composition 2 of the present disclosure. The blood was taken, and the serum was isolated. Titers of IgG antibodies against novel coronavirus S protein in machin serum were detected by the ELISA method.

**Table 14. Laboratory Animals Grouping**

| Samples | Administration Dosage | Administration Mode | Immunization Time | Number |
|---|---|---|---|---|
| pharmaceutical composition 2 (low dosage antigen 10 µg) of the present disclosure | 1 ml per machin | *i.m.* | immunizing once on days 1, 8, 15, and 29 | 5 |
| pharmaceutical composition 2 (high dosage antigen 30 µg) of the present disclosure | 3 ml per machin | *i.m.* | immunizing once on days 1, 8, 15, and 29 | *5* |

Conclusion: The pharmaceutical composition of the present disclosure can significantly increase the titers of IgG antibodies in machin serum against novel coronavirus S protein. The specific IgG antibodies can be detected 1 week after the second time of immunization of the animals in the groups of the low dosage of the pharmaceutical composition 2 of the present disclosure and the high dosage of the pharmaceutical composition 2 of the present disclosure (D15, before the third time of administration), and the occurrence rates are 10/10 and 10/10, respectively, and the antibody titer ranges from 1:1600 to >1:102400. As the number of times of administration increases, the titers of the specific IgG antibodies of animals in the low-dosage and high-dosage groups both are visibly increased. After 4 weeks of drug discontinuance and recovery (D57), no titers of specific IgG antibodies of all animals in both the low-dosage and high-dosage groups are decreased.

### Test Example 10: The pharmaceutical composition 6 of the present disclosure can effectively induce rabbits to produce neutralizing antibodies against the novel coronavirus

Method: The rabbits were immunized with the pharmaceutical composition 6 of the present disclosure. The blood was taken, and the serum was isolated. Titers of neutralizing antibodies in the rabbit serum were detected by neutralizing test of SARS-CoV-2 wild strain.

**Table 15. Laboratory Animals Grouping**

| Sample | Administration Dosage | Administration Mode | Immunization Time | Number |
|---|---|---|---|---|
| pharmaceutical composition 6 (antigen: 600 active units) of the present disclosure | 1 ml per rabbit | *i.m.* | immunizing once on days 0 and 7 | 3 |

Conclusion: The pharmaceutical composition 6 of the present disclosure can induce the titer of neutralizing antibodies in the rabbit serum.

The pharmaceutical compositions for preventing and treating the novel coronavirus SARS-CoV-2 infection provided by the present disclosure can achieve the following effects.
1. The pharmaceutical effect is better and the efficacy is significant. The ingredients of the pharmaceutical compositions of the present disclosure can activate interferon regulatory factor 3 and NF-kB, upregulate the expression of I-type interferon and proinflammatory cytokines, activate natural immunity and acquired immunity of the human body, and play an important role in resisting virus. A plurality of animal test results show that the ingredients of the pharmaceutical compositions of the present disclosure can effectively reduce the titer and load of the respiratory tract viruses of animals.
2. The pharmaceutical compositions of the present disclosure can simultaneously achieve the preventive and therapeutic effects.
3. The dosage form is flexible, the injection dosage form may be adopted, the action is rapid and reliable, the medicine liquid can be directly injected into tissues, without being affected by pH, enzyme, food, etc., and without first pass effect, and the drug content is not easy to lose, therefore, the pharmaceutical compositions of the present disclosure have reliable efficacy and can be used to rescue critical patients. The spray preparation also can be adopted, which is convenient for administration, easy to be accepted by patients, and rapidly absorbed through mucosa, takes effect quickly, and has good patient compliance.

## Claims

1. An immune composition, containing:
polyriboinosinic-polyribocytidylic acid (PIC),
an antibiotic or a polyamino compound,
a positive ion, and
optionally, an antigen derived from coronavirus, wherein
the coronavirus is SARS-CoV-2 or a variant thereof;
preferably, the antibiotic is one selected from the group consisting of tobramycin, anthracycline, butyrosin sulfate, gentamicin, hygromycin, amikacin, kanamycin, nebramycin, β-lactam, metrzamide, neomycin, puromycin, streptomycin and streptozotocin, or a combination therefrom;
preferably, the polyamino compound is one selected from the group consisting of spermidine acid salt, spermidine, N-(3-aminopropyl), N-(3-aminopropyl)-1,4-butanediamine, spermine, spermine, OS-dimethylamidothiophosphate, polylysine and aminoglycoside, or a combination therefrom;
preferably, the positive ion is a divalent positive ion, and more preferably, the positive ion is one selected from the group consisting of calcium, cadmium, lithium, magnesium, cerium, cesium, chromium, cobalt, deuterium, gallium, iodine, iron and zinc, or a combination therefrom; and most preferably, the positive ion is a calcium ion; and
preferably, the antigen derived from coronavirus is one selected from the group consisting of an inactivated virus SARS-CoV-2, an attenuated virus SARS-CoV-2, a virus SARS-CoV-2 that cannot be propagated in a subject, an S protein of SARS-CoV-2 or an immunogenic fragment thereof, an M protein or an immunogenic fragment thereof, an N protein or an immunogenic fragment thereof, an E protein or an immunogenic fragment thereof, and a protein, a polypeptide, RNA, and DNA designed according to an SARS-CoV-2 structure, or a combination therefrom.

2. The immune composition according to claim 1, wherein
when the antigen derived from coronavirus is an inactivated virus SARS-CoV-2, an attenuated virus SARS-CoV-2, or a virus SARS-CoV-2 that cannot be propagated in a subject, a ratio of the antigen derived from coronavirus to the PIC is any one selected from the group consisting of:
1 active unit/50 µg, 1 active unit/60 µg, 1 active unit/70 µg, 1 active unit/80 µg, 1 active unit/90 µg, 1 active unit/100 µg, 1 active unit/125 µg, 1 active unit/200 µg, 1 active unit/250 µg, 1 active unit/300 µg, 1 active unit/350 µg, 1 active unit/400 µg, 1 active unit/450 µg, 1 active unit/500 µg, 1 active unit/550 µg, 1 active unit/600 µg, 1 active unit/700 µg, 1 active unit/800 µg, 1 active unit/1000 µg, 1 active unit/1500 µg, 1 active unit/2000 µg, 1 active unit/2500 µg, 1 active unit/3000 µg, 1 active unit/4000 µg, 1 active unit/5000 µg, 1 active unit/6000 µg, 1 active unit/7000 µg, 1 active unit/8000 µg, 1 active unit/9000 µg 1 active unit/10000 µg and a range between any two values of the foregoing; or when the antigen derived from coronavirus is any one selected from the group consisting of an S protein of SARS-CoV-2 or an immunogenic fragment thereof, an M protein or an immunogenic fragment thereof, an N protein or an immunogenic fragment thereof, an E protein or an immunogenic fragment thereof, and a protein, a polypeptide, DNA, and RNA designed according to a SARS-CoV-2 structure, a ratio of the antigen to the PIC is any one selected from the group consisting of:
1 µg/10 µg , 1 µg/20 µg, 1 µg/30 µg, 1 µg/40 µg, 1 µg/50 µg, 1 µg/60 µg, 1 µg/70 µg, 1 µg/80 µg, 1 µg/90 µg, 1 µg/100 µg, 1 µg/125 µg, 1 µg/200 µg, 1 µg/250 µg, 1 µg/300 µg, 1 µg/350 µg, 1 µg/400 µg, 1 µg/450 µg, 1 µg/500 µg, 1 µg/550 µg, 1 µg/600 µg, 1 µg/700 µg, 1 µg/800 µg, 1 µg/1000 µg, 1 µg/1500 µg, 1 µg/2000 µg, 1 µg/2500 µg, 1 µg/3000 µg, 1 µg/4000 µg, 1 µg/5000 µg, 1 µg/6000 µg, 1 µg/7000 µg, 1 µg/8000 µg, 1 µg/9000 µg, 1 µg/10000 µg and a range between any two values of the foregoing.

3. The immune composition according to claim 1, wherein
a concentration of the PIC in the immune composition is 250 µg/unit dose to 5000 µg/unit dose; and
preferably, a concentration of the PIC in the immune composition is any one selected from the group consisting of 250 µg/unit dose, 500 µg/unit dose, 1000 µg/unit dose, 1500 µg/unit dose, 2000 µg/unit dose, 3000 µg/unit dose, 4000 µg/unit dose and 5000 µg/unit dose.

4. The immune composition according to claim 1, wherein
when the antigen derived from coronavirus is an inactivated virus SARS-CoV-2, an attenuated virus SARS-CoV-2, or a virus SARS-CoV-2 that cannot be propagated in a subject, a concentration of the antigen derived from coronavirus in the immune composition is 0.1 active unit/unit dose to 100.0 active unit/unit dose,
preferably, a concentration of the antigen derived from coronavirus in the immune composition is any one selected from the group consisting of 0.5 active units/unit dose, 1.0 active unit/unit dose, 1.5 active units/unit dose, 2.0 active units/unit dose, 2.5 active units/unit dose, 3.0 active units/unit dose, 3.5 active units/unit dose, 4.0 active units/unit dose, 5.0 active units/unit dose, 6.0 active units/unit dose, 7.0 active units/unit dose, 8.0 active units/unit dose, 9.0 active units/unit dose, 10.0 active units/unit dose, 15.0 active units/unit dose, 20.0 active units/unit dose, 30.0 active units/unit dose, 40.0 active units/unit dose, 50.0 active units/unit dose, 60.0 active units/unit dose, 70.0 active units/unit dose, 80.0 active units/unit dose, 90.0 active units/unit dose, 100.0 active units/unit dose and a range between any two values of the foregoing; or
when the antigen derived from coronavirus is any one selected from the group consisting of an S protein of SARS-CoV-2 or an immunogenic fragment thereof, an M protein or an immunogenic fragment thereof, an N protein or an immunogenic fragment thereof, an E protein or an immunogenic fragment thereof, and a protein, a polypeptide, DNA, and RNA designed according to a SARS-CoV-2 structure, a concentration of the antigen derived from coronavirus in the immune composition is 0.1 µg/unit dose to 1000.0 µg/unit dose,
preferably, a concentration of the antigen derived from coronavirus in the immune composition is any one selected from the group consisting of 0.5 µg/unit dose, 1.0 µg/unit dose, 2.0 µg/unit dose, 3.0 µg/unit dose, 4.0 µg/unit dose, 5.0 µg/unit dose, 6.0 µg/unit dose, 7.0 µg/unit dose, 8.0 µg/unit dose, 9.0 µg/unit dose, 10.0 µg/unit dose, 15.0 µg/unit dose, 20.0 µg/unit dose, 30.0 µg/unit dose, 40.0 µg/unit dose, 50.0 µg/unit dose, 60.0 µg/unit dose, 70.0 µg/unit dose, 80.0 µg/unit dose, 90.0 µg/unit dose, 100.0 µg/unit dose, 200.0 µg/unit dose, 300.0 µg/unit dose, 400.0 µg/unit dose, 500.0 µg/unit dose, 600.0 µg/unit dose, 700.0 µg/unit dose, 800.0 µg/unit dose, 900.0 µg/unit dose, 1000.0 µg/unit dose and a range between any two values of the foregoing.

5. The immune composition according to claim 3 or 4, wherein the unit dose is any one selected from the group consisting of 0.1 ml, 0.15 ml, 0.2 ml, 0.5 ml, 1.0 ml, 1.5 ml, 2.0 ml, 2.5 ml, 3.0 ml, 4.0 ml, 5.0 ml, 10.0 ml, 20.0 ml, 30.0 ml, 40.0 ml, 50.0 ml, 60.0 ml, 70.0 ml, 80.0 ml, 90.0 ml, 100.0 ml, 150.0 ml, 200.0 ml, 250.0 ml and a range between any two values of the foregoing.

6. The immune composition according to any one of claims 1 to 5, further containing one selected from the group consisting of:
gelatin, sucrose, white granulated sugar, lactose, maltose, trehalose, glucose, low molecular dextran, sorbitol, polysorbate 20, polysorbate 80, arginine hydrochloride, mannitol polyethylene glycol, human serum albumin, recombinant albumin, sodium caprylate, urea, aluminium hydroxide, aluminium phosphate, squalene, saponin, oligonucleotide, phenol red, magnesium chloride, potassium chloride, sodium chloride, sodium thiosulfate, potassium dihydrogen phosphate, ascorbic acid, chloroform, phenol and thimerosal, or a combination therefrom.

7. The immune composition according to any one of claims 1 to 6, further containing a physiologically acceptable buffer solution, which is one selected from the group consisting of acetate, trishydroxymethylaminomethane, bicarbonate, carbonate and phosphate buffer solution, or a combination therefrom;
preferably, the buffer solution has a concentration of 5 mM to 50 mM, preferably 5 mM to 20 mM; and
preferably, a pH value of the immune composition is 6 to 9.

8. The immune composition according to any one of claims 1 to 7, wherein the immune composition can be prepared in a solid dosage form or a liquid dosage form, wherein
the liquid dosage form is any one selected from the group consisting of injectable solution, suspension, spray, aerosol, naristillae, ointment, emulsion, drop, syrup and gel; and
the solid dosage form is any one selected from the group consisting of dry powder, lyophilized powder, tablet, capsule, suppository, granule and sugar-coated lozenge.

9. The immune composition according to any one of claims 1 to 8, wherein
the PIC is heterogeneous in molecular weight, and has a molecular weight of 50,000 daltons to 1,200,000 daltons; or
the PIC is heterogeneous in molecular weight, and has a sedimentation coefficient unit of 5.0 to 24.0.

10. The immune composition according to any one of claims 1 to 9, wherein
a concentration of the antibiotic or polyamino compound in the immune composition is 400 U/unit dose to 1200 U/unit dose, preferably, 400 U/unit dose, 500 U/unit dose, 600 U/unit dose, 700 U/unit dose, 800 U/unit dose, 900 U/unit dose, 1000 U/unit dose, 1100 U/unit dose, and 1200 U/unit dose; and
a concentration of the positive ion in the immune composition is 0.01 mg/unit dose to 0.1 mg/unit dose, preferably, 0.01 mg/unit dose, 0.02 mg/unit dose, 0.03 mg/unit dose, 0.04 mg/unit dose, 0.05 mg/unit dose, 0.06 mg/unit dose, 0.07 mg/unit dose, 0.08 mg/unit dose, 0.09 mg/unit dose and 0.1 mg/unit dose.

11. The immune composition according to any one of claims 1 to 10, wherein the antigen derived from coronavirus is any one selected from the group consisting of:
a SARS-CoV-2 spike protein of 2.5 µg/unit dose to 20 µg/unit dose,
a SARS-CoV-2 spike protein multimer of 2.5 µg/unit dose to 20 µg/unit dose,
an immunogenic fragment of the SARS-CoV-2 spike protein of 2.5 µg/unit dose to 20 µg/unit dose,
a SARS-CoV-2 spike protein RBD region of 2.5 µg/unit dose to 20 µg/unit dose, and
inactivated SARS-CoV-2 virion of 300 active units/unit dose to 1000 active units/unit dose, wherein
preferably, the multimer is dimer, trimer, or tetramer; and
preferably, the multimer is homomultimer.

12. The immune composition according to any one of claims 1 to 11, containing:
an antigen derived from coronavirus,
0.5 mg/ml to 2 mg/ml PIC,
400 units/ml to 1200 units/ml kanamycin,
0.01 mg/ml to 0.1 mg/ml calcium chloride,
100 mM to 200 mM sodium chloride, and
5 mM to 20 mM phosphate buffer solution, and
optionally, further containing any one selected from the group consisting of 100 mM to 200 mM arginine hydrochloride, 0.005% w/v to 0.05% w/v polysorbate 80, and 0.1% w/w to 1.0% w/w aluminium phosphate, or a combination therefrom.

13. Use of the immune composition according to any one of claims 1 to 12 in preparation of a vaccine or medicament for preventing or treating coronavirus infection, wherein
preferably, the coronavirus is SARS-CoV-2 or a variant thereof; and
preferably, the vaccine or medicament is prepared in a dosage form suitable for any one of a route of administration selected form the group consisting of intramuscular, intraperitoneal, intravenous, subcutaneous, transdermal, intradermal, oral, sublingual and respiratory tract (nose, pharynx, trachea and bronchus) administration.

14. A method for preventing or treating coronavirus SARS-CoV-2 infection, comprising:
administering to a subject a prophylactically or therapeutically effective amount of the immune composition according to any one of claims 1 to 12, wherein
preferably, the immune composition is administered to the subject at a following frequency:
1 to 4 times every four years, 1 to 3 times every three years, 1 to 2 times every two years, once a year, twice a year, 3 times a year, 4 times a year, 5 times a year, 6 times a year, once a month, twice a month, 3 times a month, 4 times a month, 5 times a month, 6 times a month, 7 times a month, 8 times a month, once a week, twice a week, 3 times a week, 4 times a week, 5 times a week, 6 times a week, once every 3 days, twice every 3 days, 3 times every 3 days, once every 2 days, twice every 2 days, once a day, and twice a day;
a time interval between the administering is the same or different; and
a route of the administering is any one selected from the group consisting of intramuscular, intraperitoneal, intravenous, subcutaneous, transdermal, intradermal, oral, sublingual and respiratory tract (nose, pharynx, trachea and bronchus) administration.
